# EUROPÄISCHE PATENTSCHRIFT

(11) **EP 4 085 886 B1**
(45) Veröffentlichungstag und Bekanntmachung des Hinweises auf die Patenterteilung: **12.02.2025**
(21) Anmeldenummer: 22163411.6
(22) Anmeldetag: 22.03.2022
(51) Int. Cl.: A61F 13/58, A61F 13/62, A61F 13/514

(54) **WEGWERFBARE WINDEL**
DISPOSABLE DIAPER
COUCHE JETABLE

(30) Priorität: 04.05.2021 DE 102021111498
(43) Veröffentlichungstag der Anmeldung: 09.11.2022
(73) Patentinhaber: LK Mahnke GmbH & Co., KG, 45479 Mühlheim and der Ruhr (DE)
(72) Erfinder: Haas, Alexandra, 42781 Haan (DE); Paz, Rui Miguel, 47800 Krefeld (DE)
(74) Vertreter: Gottschald Patentanwälte Partnerschaft mbB

(56) Entgegenhaltungen:
- EP-A1- 0 321 232
- EP-B9- 1 635 752
- DE-A1- 102006 038 334
- DE-T2- 69 619 172
- DE-T2- 69 808 629
- DE-U1- 202018 105 751
- DE-U1- 29 711 430

## Beschreibung

Die Erfindung betrifft eine wegwerfbare Windel, insbesondere Babywindel, nach dem Oberbegriff des Anspruchs 1.

Unter wegwerfbaren Windeln, auch als Wegwerfwindel oder Einwegwindel bezeichnet, werden körpernah eingesetzte saugfähige Produkte, insbesondere Babywindeln, aber auch Erwachsenenwindeln, Inkontinenzwindeln oder dergleichen verstanden. Diese weisen ein flüssigkeitsdurchlässiges, körperseitiges Deckblatt (Topsheet) und ein flüssigkeitsundurchlässiges, meist atmungsaktives, körperfernes Rückenblatt (Backsheet) auf, zwischen denen üblicherweise eine flüssigkeitsabsorbierende Sauganordnung mit einem Saugkörper und einer Verteilerlage angeordnet ist. Der Schichtaufbau mit Deckblatt, Sauganordnung und Rückenblatt weist seitliche Befestigungslaschen auf, die ein Trägermaterial, insbesondere eine Trägerfolie, aufweisen, auf der ein Klettmaterial bildende Hakenbereiche angeordnet sind. Zum Befestigen der Windel am Körper des Benutzers werden die Befestigungslaschen über ihre Hakenbereiche mit einer zugeordneten Befestigungsfläche (Komfortbündchen) auf dem Rückenblatt zusammengebracht, wodurch eine Klettverbindung gebildet wird. Die mit dem jeweiligen Hakenbereich zusammenwirkende Befestigungsfläche wird von einem Bereich des Rückenblatts, der mit einer Vliesschicht versehen ist, gebildet, in welchem sich der jeweilige Hakenbereich verhaken kann.

Die bekannte Windel (DE 20 2018 105 751 U1), von der die Erfindung ausgeht, weist Befestigungslaschen mit Hakenbereichen und Kleberbereichen auf, die im Haltezustand bzw. im bestimmungsgemäß am Körper des Benutzers befestigen Zustand der Windel jeweils mit der Vliesschicht des Rückenblatts in Kontakt kommen. Die Hakenbereiche werden dabei von einem quer zu dessen Erstreckungsrichtung geschlitzten und zu einem Streckgitter auseinandergezogenen Hakenband gebildet, wobei die einzelnen Stege des Streckgitters jeweils mehrere Haken aufweisen. In den Gitterzwischenräumen befinden sich die zugänglichen Kleberbereiche, die von einer Kleberschicht gebildet werden, über die das den jeweiligen Hakenbereich bildende Streckgitter an der Trägerfolie der Befestigungslasche befestigt ist.

Bei der herkömmlichen Windel haben sich Kombinationen aus Hakenbereichen und etwa 45% Kleberbereichen an der Befestigungslasche als vorteilhafte Kombination herausgestellt. Allerdings ist der Herstellungsprozess des Streckgitters relativ aufwendig. Zusätzlich soll zur Schonung der Umwelt weniger Kleber verwendet werden. Ein weiterer Nachteil von Kleber ist, dass sich dieser mit der Zeit abnutzt, so dass die Anzahl an Wiederverschließvorgängen, die bei der Windel möglich sind, begrenzt ist.

Der Erfindung liegt das Problem zugrunde, eine wegwerfbare Windel der eingangs genannten Art derart auszugestalten und weiterzubilden, dass eine stabile Befestigung zwischen Befestigungslasche und Vliesschicht kostengünstiger und herstellungstechnisch einfacher erreicht wird.

Es ist nun vorliegend gelungen, mit weniger als 20% Kleberbereichen eine ausreichende Scherfestigkeit und somit ein gutes Halteverhalten zu erzeugen.

Dieser Entwicklung liegen diverse Erkenntnisse zugrunde. Zum einen hat sich die Herstellung eines Streckgitters mit weniger als 20% Kleberanteilen als mechanisch kompliziert erwiesen und zum anderen erscheint es wenig intuitiv, ein Streckgitter kaum zu strecken, sodass die Zwischenräume des Streckgitters flächenmäßig unter 20% betragen. Versuche mit einer rechteckigen Hakenplatte, die außen von Kleber gesäumt war, haben hingegen zum sogenannten Fiberpicking geführt. Dabei werden einzelne Fasern aus der Vliesschicht des Rückblattes herausgerupft, die an der Befestigungslasche hängen bleiben und deren Haltekraft bei einem Wiederverschließen deutlich reduzieren.

Es hat sich bei Versuchen mit anderen Geometrien der Hakenbereiche gezeigt, dass es vor allem darauf ankommt, die Hakenbereiche zumindest teilweise voneinander zu beabstanden, indem mindestens ein hakenfreier Bereich zwischen den Hakenbereichen angeordnet wird.

Durch die geschickte Kombinierung von Hakenbereichen und mindestens einem hakenfreien Bereich kann erreicht werden, dass gleichzeitig die notwendige Menge an Haken und ggfs. Kleber auf dem Rückblatt aufliegt, um genügend Haltekraft aufzubringen und nicht zu viele Haken an einer einzelnen Vliesfaser angreifen. Damit lässt sich bei geeigneter Auslegung auch der Einsatz von Kunststoff für die Hakenbereiche reduzieren, was mit Blick auf die Schonung der Umwelt besonders vorteilhaft ist.

Wesentlich ist somit die grundsätzliche Überlegung, dass nur eine gewisse Haltekraft zwischen einer einzelnen Faser des Vlieses und mehreren Haken möglich ist, bevor die Haken die Faser ausreißen. Dabei hat sich außerdem gezeigt, dass Kleberbereiche ein anderes Löseverhalten von den Vliesfasern aufweisen und dieses Problem gerade nicht verstärken. Es wird somit möglich, einen Großteil der Haltekraft zwischen Befestigungslasche und Rückenblatt durch Hakenbereiche aufzubringen, in dem diese so verteilt werden, dass sich die Haltekraft auf genügend unterschiedliche Fasern des Vlieses verteilt.

Diese Erkenntnis ermöglicht es nun, weniger als 20% Kleberbereiche auf der Befestigungslasche vorzusehen, ohne die Haltefunktion der Befestigungslasche zu beeinträchtigen.

Im Einzelnen wird vorgeschlagen, dass die Befestigungsfläche flächig außerhalb der Hakenbereiche höchstens Kleberbereiche aufweist, die im Haltezustand weniger als 20% der von der Befestigungsfläche überdeckten, ungebondeten Bereiche überdecken, und dass mindestens ein hakenfreier Bereich der Befestigungslasche, insbesondere ein Kleberbereich, zwischen den Hakenbereichen angeordnet ist.

Geringe Mengen an Kleber haben sich als vorteilhaft herausgestellt. Anspruch 2 betrifft daher bevorzugte Kombinationen zwischen Hakenbereichen und Kleberbereichen in Prozent.

Bei einer Ausgestaltung gemäß Anspruch 3 können mehrere voneinander beabstandete und/oder voneinander getrennte Hakenbereiche vorgesehen sein. Das Aufkleben mehrerer Hakenbereiche ist produktionstechnisch sehr einfach umzusetzen.

Formen und Größen der Hakenbereiche und des mindestens einen hakenfreien Bereichs, die eine gute Haltewirkung aufweisen, sind Gegenstand der Ansprüche 4 bis 6. Bevorzugte Gesamtbreiten der Befestigungsfläche und/oder der Hakenbereiche sind Gegenstand von Anspruch 7. Bei üblichen Vliesmaterialien und insbesondere in Kombination mit den bevorzugten Bondingmustern ermöglichen diese Größen und Formen hohe Haltekräfte unter Vermeidung von relevanten Mengen an Fiberpicking, die eine Wiederverschließbarkeit der Windel zu stark reduzieren würden.

Gemäß Anspruch 9 nimmt das Bondingmuster vorzugsweise zumindest den größten Teil der Fläche der Vliesschicht und/oder die Vliesschicht zumindest den größten Teil der Fläche des Rückenblatts ein. Gegenüber der Verwendung von Komfortbündchen ergibt sich so die Möglichkeit der freien Platzierung der Befestigungslaschen am Rückenblatt.

Die Ansprüche 10 bis 14 betreffen bevorzugte Ausgestaltungen des Bondingmusters. Gemäß Anspruch 10 können die ungebondeten Bereiche regelmäßige Musterelemente ergeben. Grundsätzlich hat sich gezeigt, dass es für die Haltewirkung der Befestigungslasche und insbesondere der Haken notwendig ist, dass eine hinreichende Menge an Fasern der Vliesschicht des Rückenblattes durch die Bondingmusterelemente gehalten wird. Regelmäßige Bondingmuster, insbesondere nach den Ausgestaltungen gemäß den Ansprüchen 11 und 12, haben sich dafür als besonders geeignet hervorgetan. Dabei sind insbesondere die ungebondeten Bereiche wirksam für das Einhaken der Haken und die gebondeten Bereiche halten die so eingehakten Fasern in der Vliesschicht.

Aufgrund der Belastung der Befestigungslasche vor allem entlang einer Gürtelrichtung des Benutzers ist es vorzugsweise bei einer Ausgestaltung gemäß Anspruch 13 so, dass jedenfalls in einer Richtung quer zu dieser Hauptzugrichtung diverse Fasern ein- oder zweimal gebondet sind. Unter Beanspruchung spannen sich diese Fasern ähnlich wie eine Bogensehne und halten so die Befestigungslasche am Rückenblatt. Anspruch 14 weitet die Überlegungen von Anspruch 13 dabei auch auf die anderen Richtungen aus, in denen die Befestigungslasche selbstverständlich auch eine gewisse Haltekraft aufbringen muss.

Anspruch 15 spezifiziert, dass die Vliesschicht, vorzugsweise auf einem Vliesschicht-Trägermaterial, insbesondere einer Folie, aufgebracht ist. Somit wird der bevorzugte Anwendungsfall weiter eingegrenzt.

Die Ansprüche 16 und 17 betreffen bevorzugte Ausgestaltungen der Vliesschicht und insbesondere der Faser.

Neben der Scherbelastung ist sowohl für das Halten als auch eine Benutzerfreundlichkeit die maximale Peel-Kraft relevant. Anspruch 18 gibt bevorzugte Werte dieser Peel-Kraft an.

Im Folgenden wird die Erfindung anhand einer lediglich ein Ausführungsbeispiel darstellenden Zeichnung näher erläutert. In der Zeichnung zeigt
- Fig. 1: in einer schematischen Perspektivansicht eine vorschlagsgemäße wegwerfbare Windel,
- Fig. 2: in einer Draufsicht mit übereinstimmenden Größenverhältnissen in a) einen Ausschnitt eines Bondingmusters der Vliesschicht der Windel gemäß Fig. 1 und in b) einen Ausschnitt einer Befestigungslasche einer Windel gemäß Fig. 1,

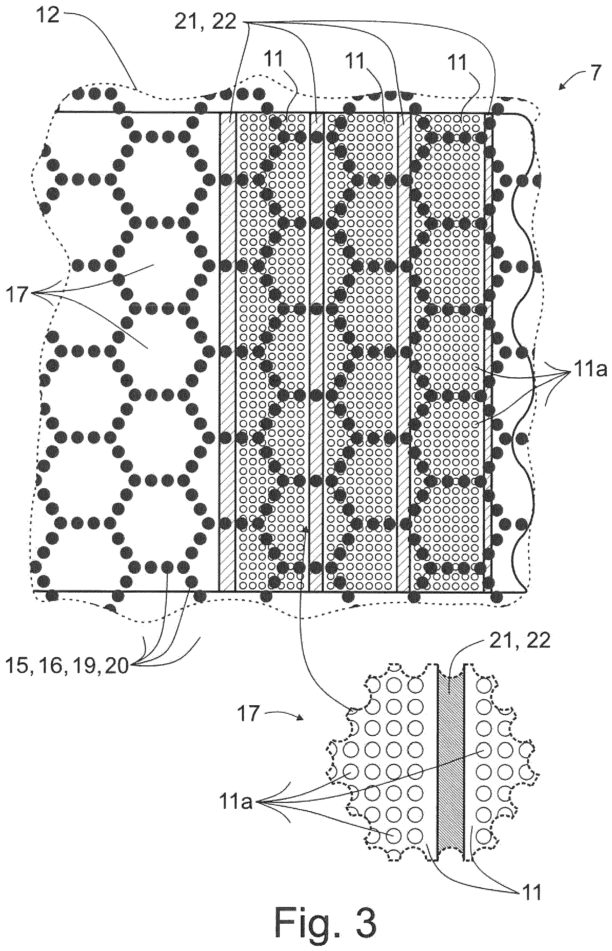

- Fig. 3: in einer übereinandergelegten Ansicht die Vliesschicht mit dem Bondingmuster und die Befestigungslasche gemäß Fig. 2,
- Fig. 4: eine schematische Erläuterung des Bogensehen-Effekts, der eine erhöhte Scherfestigkeit hervorruft,
- Fig. 5: diverse bevorzugte Hakenformen des Hakenbereiches und
- Fig. 6: diverse bevorzugte Bondingmuster der Vliesschicht.

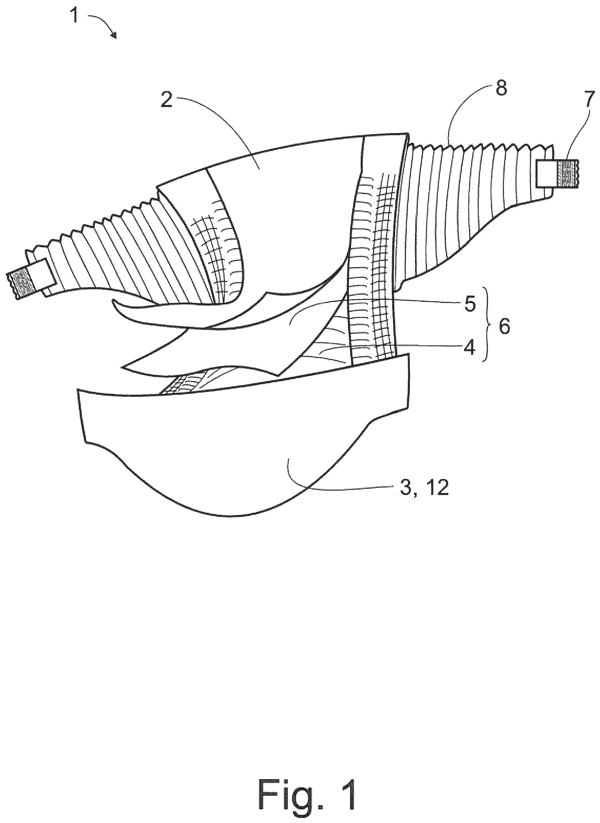

In der perspektivischen Ansicht gemäß Fig. 1 ist beispielhaft eine wegwerfbare Windel 1, hier in Form einer Babywindel, dargestellt. Grundsätzlich kann es sich bei der Windel aber auch um eine Erwachsenenwindel oder Inkontinenzwindeln handeln. Die vorschlagsgemäße Windel 1 weist ein hier und vorzugsweise flüssigkeitsdurchlässiges, körperseitiges Deckblatt 2, auch Topsheet genannt, und ein hier und vorzugsweise flüssigkeitsundurchlässiges, körperfernes Rückenblatt 3, auch Backsheet genannt, auf. Zwischen dem Deckblatt 2 und dem Rückenblatt 3 sind weitere Lagen in dem Schichtaufbau vorgesehen, nämlich ein Saugkörper 4 und eine Verteilerlage 5, die zusammen eine flüssigkeitsabsorbierende Sauganordnung 6 bilden. Ferner weist die Windel mindestens eine Befestigungslasche 7, hier genau zwei Befestigungslaschen 7, auf, die an Seitenflügeln 8 der Windel 1 angeordnet sind. Die Seitenflügel 8 sind mit dem Schichtaufbau der Windel, insbesondere dem Rückenblatt 3, verbunden, insbesondere verklebt, oder einstückig damit ausgeführt. Die jeweilige Befestigungslasche 6 ist insbesondere mit dem zugeordneten Seitenflügel 8 verbunden, insbesondere verklebt, oder einstückig damit ausgeführt.

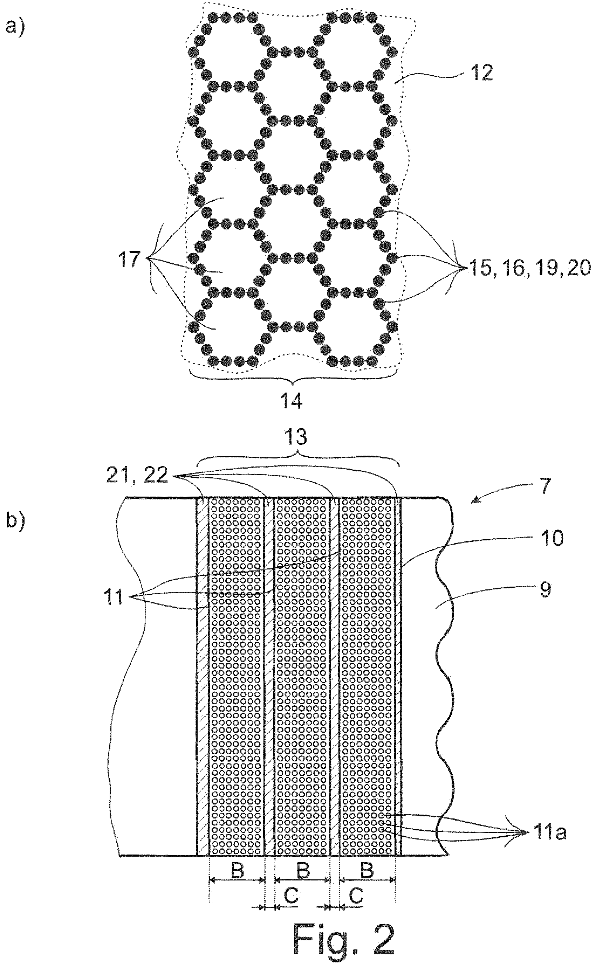

Die jeweilige Befestigungslasche 7 weist, wie Fig. 2b) veranschaulicht, hier und vorzugsweise mehrere Lagen auf, von denen eine ein Trägermaterial 9, insbesondere in Form einer Folie, bildet, wobei auf dem Trägermaterial 9 eine hier und vorzugsweise durchgehende Kleberschicht 10 und darauf wiederum Hakenbereiche 11, vorzugsweise mit einer Vielzahl von einzelnen Haken 11a, angeordnet sein können. Hier und vorzugsweise verbindet die Kleberschicht 10 die Hakenbereiche 11 stoffschlüssig mit dem Trägermaterial 9.

Als Hakenbereich wird vorliegend ganz allgemein ein sich über eine zusammenhängende Fläche der Befestigungslasche 7 erstreckender Bereich verstanden, der sich zumindest am Rückenblatt 2 über eine Vielzahl von Hakenverbindungen verhaken kann. Hierfür weist er vorzugsweise eine Vielzahl einzelner, oben genannter Haken 11a auf, die sich über den Hakenbereich verteilen. Weiter vorzugsweise erstrecken sich die Vielzahl von Haken 11a jeweils separat, ausgehend von einem flächigen Substrat des Hakenbereichs aus Kunststoff. Das Substrat kann insbesondere, wie oben angesprochen, stoffschlüssig mit dem Trägermatieral 9 der Befestigungslasche 7 verbunden sein.

Eine obige Befestigungslasche 7 wird zur bestimmungsgemäßen Befestigung der Windel am Körper des Benutzers mit dem Rückenblatt 2, und zwar insbesondere dessen in Fig. 1 nach vorne gerichtetem Teil des Rückenblatts 2, der dann als Komfortbündchen fungiert, zusammengeführt. Zur Verbindung mit der Befestigungslasche 7 weist das Rückenblatt 2 an seiner vom Deckblatt 1 abgewandten Seite (Windelaußenseite) eine Vliesschicht 12 auf, die sich hier und vorzugsweise zumindest über den größten Teil der Fläche des Rückenblatts 3, hier insbesondere die gesamte Fläche des Rückenblatts 3, erstreckt. Die Befestigungslasche 7 ist somit an jeder Stelle des Rückenblatts 3 mit diesem in Kontakt bringbar.

Konkret weist die vorschlagsgemäße wegwerfbare Windel 1 zumindest ein körperseitiges Deckblatt 2, ein körperfernes Rückenblatt 3 und mindestens einen Seitenflügel 8 auf, wobei an dem Seitenflügel 8 eine Befestigungslasche 7 angeordnet ist und wobei die Befestigungslasche 7 eine Befestigungsfläche 13 mit Hakenbereichen 11 aufweist.

Vorschlagsgemäß weist das Rückenblatt 3 die Vliesschicht 12, auch Nonwoven-Schicht genannt, auf. Die Vliesschicht 12 ist vorschlagsgemäß in einem Bondingmuster 14 aus Bondingmusterelementen 15 gebondet, also verfestigt, und so in gebondete Bereiche 16 und ungebondete Bereiche 17 unterteilt. Hier und vorzugsweise ist die Vliesschicht 12 zusammen mit einem Vliesschicht-Trägermaterial, das beispielsweise in Form einer Folie vorliegt, gebondet, hier und vorzugsweise punktförmig gebondet, was beispielsweise in einem Kalander unter erhöhtem Druck und erhöhter Temperatur durchgeführt worden ist. Durch das Bonden werden die die Vliesschicht 12 bildenden Fasern 18 komprimiert und zumindest miteinander und gegebenenfalls auch mit dem Vliesschicht-Trägermaterial verschmolzen bzw. verschweißt, wodurch Konturen in der Vliesschicht 12 erzeugt werden, die die Bondingmusterelemente 15 bilden, die zusammen wiederrum das Bondingmuster 14 bilden. An den Stellen, an denen die Vliesschicht 12 gebondet worden ist, entstehen sogenannte Prägeelemente 19, bei dem hier punktförmigen Bonden Prägepunkte 20, hier beispielhaft mit kreisförmiger Querschnittsform. Fig. 2a) zeigt eine Vielzahl solcher Prägepunkte 20.

Die Befestigungsfläche 13 ist für die Einnahme eines Haltezustands derart an dem Rückenblatt 3 befestigbar, dass die Befestigungsfläche 13 gebondete und ungebondete Bereiche 16, 17 überdeckt. Dieser Haltezustand wird im bestimmungsgemäßen Gebrauch eingenommen, wenn die Windel 1 einem Benutzer angelegt ist.

Mit dem "Bonden" ist hier ein Verfestigen der Vliesschicht durch Verprägen gemeint, was im Folgenden noch näher erläutert wird. Die Vliesschicht 12, die auf diese Weise verfestigt wird, kann selbst auch schon mechanisch, chemisch und/oder thermisch vorverfestigt worden sein, beispielsweise wasserstrahlverfestigt oder thermoverfestigt. Die Vliesschicht 12, die dem Verfahrensschritt des Bondens durch Verprägen und/oder dem Verfahrensschritt des Vorverfestigens ausgesetzt worden ist, kann wiederum von einem Vlies aus einer Polymerschmelze (Spinnvlies), von einem nassgelegten Vlies (wetlaid) oder von einem trockengelegten Vlies (drylaid), insbesondere von einem mechanisch trockengelegten Vlies (carded) oder von einem aerodynamisch trockengelegten Vlies (airlaid), gebildet worden sein.

Durch das Bonden wird die Vliesschicht 12 an einer Vielzahl von Stellen unter Anwendung erhöhten Drucks und erhöhter Temperatur verprägt, das heißt verschweißt, wodurch eine Verfestigung der Vliesschicht 12 erzeugt wird. Ein solches Bonden wird beispielsweise in einem Prägekalander oder in einer Ultraschall-Schweißanlage durchgeführt, um nur einige Beispiele zu nennen. An den Stellen, an denen das Verprägen bzw. Verschweißen erfolgt, werden Prägeelemente 19, beispielsweise Prägepunkte 20 und/oder Prägelinien, erzeugt, die zusammen ein Bondingmuster 14 bilden. Im Bereich der Prägeelemente 19 ist das Fasermaterial der Vliesschicht 12 komprimiert, wohingegen es an den übrigen Stellen ein größeres Volumen einnimmt. An diesen übrigen Stellen, die gegenüber der Ebene, entlang der das Rückenblatt verläuft, weiter hervorstehen, erfolgt dann im Haltezustand der Kontakt mit der Befestigungsfläche 13. Dabei ist es denkbar, die Vliesschicht 12 allein oder zusammen mit einem Vliesschicht-Trägermaterial, beispielsweise in Form einer Folie, einem Gewebe oder einer weiteren Vliesschicht, zu bonden. Wird die Vliesschicht 12 nicht zusammen mit einem Vliesschicht-Trägermaterial gebondet, kann die Vliesschicht 12 zur Bildung des Rückenblatts auch nach dem Bonden mit einem Vliesschicht-Trägermaterial, beispielsweise in Form einer Folie, einem Gewebe oder einer weiteren Vliesschicht, verklebt werden. Grundsätzlich kann die Vliesschicht auch allein das Rückenblatt bilden.

Durch das Bonden werden nun mehrere Fasern 18 im Bereich des jeweiligen Prägeelements 19, beispielsweise des jeweiligen Prägepunkts 20, miteinander und gegebenenfalls mit dem Vliesstoff-Trägermaterial verschmolzen und dadurch an dieser Stelle fixiert. Von diesen im Bereich der Prägeelemente 19 fixierten Fasern erstrecken sich mehrere auch in die übrigen, nicht-gebondeten bereiche 17, die zum Kontakt mit den Hakenbereichen 11 dienen.

Wesentlich ist nun, dass die Befestigungslasche 7 flächig außerhalb der Hakenbereiche 11 höchstens Kleberbereiche 21 aufweist, die im Haltezustand weniger als 20% der von der Befestigungsfläche 13 überdeckten, ungebondeten Bereiche 17 überdecken. Im einfachsten Fall weist die Befestigungslasche 7 keine Kleberbereiche 21 auf. Aufgrund der freien Platzierbarkeit der Befestigungslasche 7 lässt sich die Überdeckung der Klebebereiche 21 oder Hakenbereiche 11 zumeist auch ohne Betrachtung des Bondingmusters 14 schlicht über eine Betrachtung der Befestigungsfläche 13 ermitteln.

Wesentlich ist weiterhin, dass mindestens ein hakenfreier Bereich 22 der Befestigungslasche 7, insbesondere ein Kleberbereich 21, zwischen den Hakenbereichen 11 angeordnet ist. Wie im einleitenden Teil der Beschreibung bereits erläutert, lässt sich ein Fiberpicking so reduzieren oder ganz vermeiden.

Der Begriff "Befestigungsfläche" bezieht sich hier auf alle Flächen, die wirksam zur Befestigung zwischen Befestigungslasche 7 und Rückenblatt 3 bzw. Vliesschicht 12 beitragen. Dies umfasst insbesondere die Hakenbereiche 11 und ggfs. die Kleberbereiche 21 jedoch möglicherweise nicht die hakenfreien Bereiche 22, die keine Kleberbereiche 21 sind, sofern vorhanden.

Es wird darauf hingewiesen, dass die Hakenbereiche 11 auch miteinander verbunden sein können.

Es hat sich gezeigt, dass eine gewisse Fläche an Kleberbereichen 21 produktionstechnisch schwer vermeidbar ist und gleichzeitig vorteilhaft zur Haltewirkung zwischen der Befestigungslasche 7 und dem Rückenblatt 3 beiträgt. Entsprechend ist es hier und vorzugsweise so, dass die Befestigungslasche 7 flächig außerhalb der Hakenbereiche 11 Kleberbereiche 21 aufweist, die im Haltezustand mindestens 1%, vorzugsweise mindestens 4%, weiter vorzugsweise mindestens 8%, der von der Befestigungsfläche 13 überdeckten, ungebondeten Bereiche 17 überdecken. Aus Gründen der Nachhaltigkeit und zur einfacheren Produktion sollen die Kleberbereiche 21 jedoch begrenzt werden. Hier und vorzugsweise weist die Befestigungslasche 7 flächig außerhalb der Hakenbereiche 11 höchstens Kleberbereiche 21 auf, die im Haltezustand weniger als 19,5%, vorzugsweise weniger als 19%, weiter vorzugsweise weniger als 17%, noch weiter vorzugsweise weniger als 15% und noch weiter vorzugsweise weniger als 12% der von der Befestigungsfläche 13 überdeckten, ungebondeten Bereich 17 überdecken. Grundsätzlich könnten auch die Hakenbereiche 11 mit Kleber versehen sein, der dann jedoch nicht in die Berechnung einfließen würde.

Hier und vorzugsweise besteht die Befestigungsfläche 13 ausschließlich aus Hakenbereichen 11 und Kleberbereichen 21.

Die Verbindung zwischen Befestigungslasche 7 und Vliesschicht 12 weist im Haltezustand vorzugsweise eine absolute Scherfestigkeit von mindestens 10 N, vorzugsweise von mindestens 15 N, weiter vorzugsweise von mindestens 20 N, auf. Die Scherfestigkeit wird dabei durch ein Ziehen der Befestigungslasche 7 im Winkel von 180° zur Vliesschicht 12 an der Windel 1 oder nach SGS Courtray Methode C52b bestimmt.

Wie in Fig. 2b) ersichtlich, kann vorgesehen sein, dass die Befestigungslasche 7 mindestens zwei, vorzugsweise mindestens drei, voneinander beabstandete Hakenbereiche 11 aufweist. Zusätzlich oder alternativ kann die Befestigungslasche 7 mindestens zwei, vorzugsweise mindestens drei, voneinander getrennte Hakenbereiche 11 aufweisen. Unter voneinander getrennten Hakenbereichen 11 sind solche Hakenbereiche 11 zu verstehen, die nicht einstückig ausgestaltet sind. Sie können einander dennoch berühren. Derartige Ausgestaltungen haben sich als einfach zu produzieren und dennoch effektiv herausgestellt. Entsprechend ist hier und vorzugsweise vorgesehen, dass die Hakenbereiche 11 nicht oder nicht vollständig durch ein Streckgitter gebildet werden.

Im Folgenden werden diverse geometrische Ausgestaltungen der Befestigungslasche 7 dargestellt, die sich als besonders vorteilhaft erwiesen haben.

Wie in Fig. 2b) dargestellt, kann oder können mindestens ein Hakenbereich 11, vorzugsweise mindestens zwei Hakenbereiche 11, weiter vorzugsweise mindestens drei Hakenbereiche 11, insbesondere alle Hakenbereiche 11, länglich, vorzugsweise rechteckig, oder quadratisch sein. Es darf darauf hingewiesen werden, dass der Begriff länglich weit auszulegen ist und nicht nur längliche, gerade Hakenbereiche, sondern auch längliche, gebogene Hakenbereiche umfasst. Schließlich umfasst er in einer weiteren bevorzugten Ausgestaltung auch längliche, entlang einer beliebigen Kontur, beispielsweise entlang einer Wellenkontur, verlaufende Hakenbereiche. Weitere Ausgestaltungen sind denkbar.

Die Befestigungslasche 7 ist im Haltezustand vorzugsweise entlang einer Gürtelrichtung des Benutzers so ausgerichtet, dass eine Hauptzugrichtung L entlang der Gürtelrichtung verläuft. Die Hauptzugrichtung L entspricht dabei der Richtung, in der üblicherweise die größten Kräfte zu erwarten sind. Die Hakenbereiche 11 sind vorzugsweise quer, insbesondere orthogonal, zur Hauptzugrichtung L länglich. Grundsätzlich ist es aber auch denkbar, dass die Hakenbereiche 11 längs zur Hauptzugrichtung L ausgerichtet sind.

Zusätzlich oder alternativ kann vorgesehen sein, dass mindestens ein Hakenbereich 11, vorzugsweise mindestens zwei Hakenbereiche 11, weiter vorzugsweise mindestens drei Hakenbereiche 11, insbesondere alle Hakenbereiche 11, eine maximale Breite B entlang der Hauptzugrichtung L von 8 mm, vorzugsweise 6 mm, weiter vorzugsweise 4 mm, aufweist oder aufweisen.

Weiterhin kann vorgesehen sein, dass mindestens ein Hakenbereich 11, vorzugsweise mindestens zwei Hakenbereiche 11, weiter vorzugsweise mindestens drei Hakenbereiche 11, insbesondere alle Hakenbereiche 11, eine minimale Breite B entlang der Hauptzugrichtung L von 2 mm, vorzugsweise 3 mm, weiter vorzugsweise 4 mm, aufweist oder aufweisen.

Weiterhin kann vorgesehen sein, dass mindestens zwei, vorzugsweise mindestens drei, hakenfreie Bereiche 22 der Befestigungslasche 7 zwischen den Hakenbereichen 11 angeordnet sind. In Fig. 2b) sind genau zwei hakenfreie Bereiche 22 zwischen den Hakenbereichen 11 angeordnet. Zusätzlich oder alternativ kann vorgesehen sein, dass mindestens einer, vorzugsweise mindestens zwei, der zwischen den Hakenbereichen 11 angeordneten hakenfreien Bereiche 22 vollständig von Hakenbereichen 11 umgeben ist oder sind und/oder mindestens einer, vorzugsweise mindestens zwei, der zwischen den Hakenbereichen 11 angeordneten hakenfreien Bereiche 22 auf mindestens einer, vorzugsweise mindestens zwei, Seiten nicht an einen Hakenbereich 11 grenzt, wie in Fig. 2b) gezeigt.

Hier und vorzugsweise sind mindestens ein oder mindestens zwei hakenfreie Bereiche 22 auf einer oder beiden Seiten orthogonal zur Hauptzugrichtung L nicht von Hakenbereichen 11 umgeben.

Grundsätzlich gelten alle Ausführungen zu hakenfreien Bereichen 22 vorzugsweise jeweils oder einzeln für Kleberbereiche 21, sodass jeder beliebige erwähnte hakenfreie Bereich 22 auch ein Kleberbereich 21 sein kann.

Durch die insbesondere längliche Ausgestaltung der Hakenbereiche 11 lässt sich in Richtung der Hauptzugrichtung L ein Einhaken in viele Fasern 18 der Vliesschicht 12 erreichen, während gleichzeitig beim Abziehen der Befestigungslasche 7 aufgrund der Richtung des Abziehens orthogonal zur Fläche keine Probleme auftreten. Zur Scherfestigkeit beim Ziehen in die Hauptzugrichtung L tragen dabei im wesentlichen Fasern 18 bei, die quer zur Hauptzugrichtung L verlaufen und entsprechend einigermaßen zu den Hakenbereichen 11 ausgerichtet sind.

Mindestens ein hakenfreier Bereich 22, vorzugsweise mindestens zwei hakenfreie Bereiche 22, weiter vorzugsweise mindestens drei hakenfreie Bereiche 22, insbesondere alle hakenfreien Bereiche 22, können länglich, vorzugsweise rechteckig oder quadratisch sein. Die längliche Richtung kann sich dabei ebenfalls auf eine Richtung quer, insbesondere orthogonal zur Hauptzugrichtung L beziehen.

Zusätzlich oder alternativ kann mindestens ein hakenfreier Bereich 22, vorzugsweise mindestens zwei hakenfreie Bereiche 22, weiter vorzugsweise mindestens drei hakenfreie Bereiche 22, insbesondere alle hakenfreien Bereiche 22, eine maximale Breite C entlang der Hauptzugrichtung L von 6mm, vorzugsweise 4mm, weiter vorzugsweise 2mm, aufweisen. Mindestens ein hakenfreier Bereich 22, vorzugsweise mindestens zwei hakenfreie Bereiche 22, weiter vorzugsweise mindestens drei hakenfreie Bereiche 22, insbesondere alle hakenfreien Bereiche 22, können eine minimale Breite C entlang der Hauptzugrichtung L von 0,5 mm, vorzugsweise 1 mm, weiter vorzugsweise 1,5 mm, aufweisen. Dabei sind jeweils die hakenfreien Bereiche 22 zwischen den Hakenbereichen 11 gemeint. Das außerhalb der Hakenbereiche 11 natürlich auch hakenfreie Bereiche 22 liegen, ist insofern, sofern diese nicht Teil der Befestigungsfläche 13 sind, uninteressant.

Die Befestigungsfläche 13 und/oder die Hakenbereiche 11 können eine Gesamtbreite entlang der Hauptzugrichtung L von mindestens 0,7 cm, vorzugsweise mindestens 1 cm, weiter vorzugsweise mindestens 1,2 cm und/oder eine maximale Gesamtbreite entlang der Hauptzugrichtung L von 2 cm, vorzugsweise 1,7 cm, weitervorzugsweise 1,5 cm aufweisen. Die Länge der Befestigungsfläche 13 und/oder der Hakenbereiche 11 insgesamt orthogonal zur Hauptzugrichtung L beträgt vorzugsweise mindestens 2 cm, weiter vorzugsweise mindestens 2,5 cm, noch weiter vorzugsweise mindestens 3 cm und/oder höchstens 5 cm, vorzugsweise höchstens 4 cm, weiter vorzugsweise höchstens 3,5 cm.

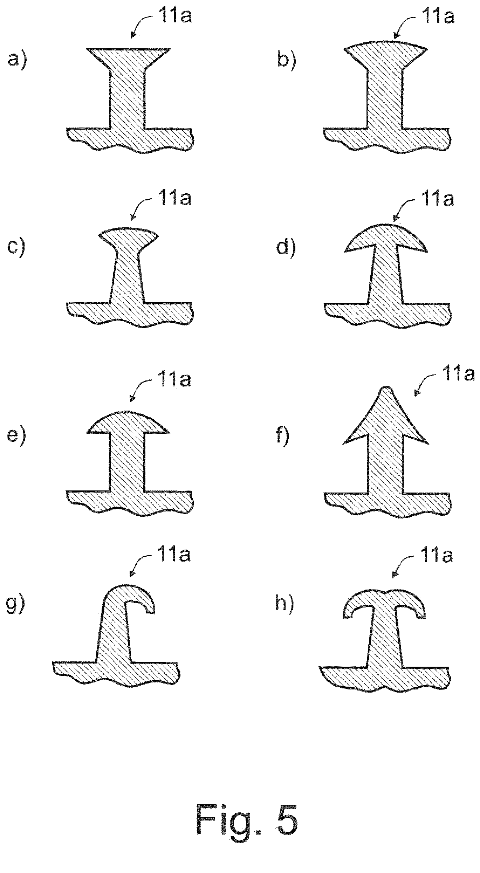

Die einzelnen Haken 11a können diverse Formen aufweisen. Bevorzugte Formen sind in Fig. 5 gezeigt. Die Haken 11a können eine Pilzform (Fig. 5c) bis 5f)), die sich durch eine gerundete Spitze auszeichnet, aufweisen. Zusätzlich oder alternativ können die Haken 11a symmetrisch um eine Längsachse sein und/oder eine Widerhakenform mit Hinterschnitt (Fig. 5d) bis 5h)) aufweisen. Als Alternative zum Hinterschnitt kann auch vorgesehen sein, dass die Haken 11a derart angeschrägt sind, dass sich das Gegenteil eines Hinterschnitts ergibt, wodurch die Fasern 18 sich leichter lösen (Fig. 5a) bis 5c)).

Insgesamt ist es hier und vorzugsweise so, dass die Hakenbereiche 11 eine Flächendichte an Haken 12 von mindestens 150 Haken pro 100 mm², vorzugsweise mindestens 200 Haken pro 100 mm², vorzugsweise mindestens 250 Haken pro 100 mm², und/oder höchstens 450 Haken pro 100 mm², vorzugsweise höchstens 400 Haken pro 100 mm², weiter vorzugsweise höchstens 350 Haken pro 100 mm², aufweisen.

Im Hinblick auf die Vliesschicht 12, die bei der Befestigung den Gegenpart zur Befestigungslasche 7 bildet, ist es hier und vorzugsweise so, dass das Bondingmuster 14 zumindest den größten Teil der Fläche der Vliesschicht 12, vorzugsweise die gesamte Fläche der Vliesschicht 12, einnimmt und/oder, dass die Vliesschicht 12 zumindest den größten Teil der Fläche des Rückenblatts 3, insbesondere die gesamte Fläche des Rückenblatts 3, einnimmt. Dadurch kann ermöglicht werden, dass die Befestigungslasche 7 an grundsätzlich jeder beliebigen Stelle des Rückenblatts 3 befestigbar wird.

Wie in Fig. 2a) ersichtlich, kann vorgesehen sein, dass die Bondingmusterelemente 15 so angeordnet sind, dass die ungebondeten Bereiche 17 regelmäßige Musterelemente ergeben, wobei vorzugsweise die Musterelemente ohne Zwischenräume angeordnet sind und/oder alle Musterelemente denselben Querschnitt aufweisen. Weiterhin kann vorgesehen sein, dass alle Musterelemente jeweils eine punktsymmetrische und/oder achsensymmetrische Außenkontur aufweisen und/oder, dass einzelne oder alle Musterelemente jeweils eine runde oder eckige, vorzugsweise hexagonale, Außenkontur aufweisen. Vorzugsweise weist das Bondingmuster 14 insgesamt eine Hexagonalstruktur auf.

Wie bereits erläutert entstehen die Bondingmusterelemente 15 vorzugsweise durch ein Prägen. Es kann vorgesehen sein, dass die Bondingmusterelemente 15 Prägeelemente 19, insbesondere zueinander, insbesondere gleichmäßig, beabstandete Prägepunkte 20 und/oder Prägelinien aufweisen. Im Bereich des jeweiligen Prägeelements 19 weist die Vliesschicht 12 vorzugsweise ihre minimale Dicke auf.

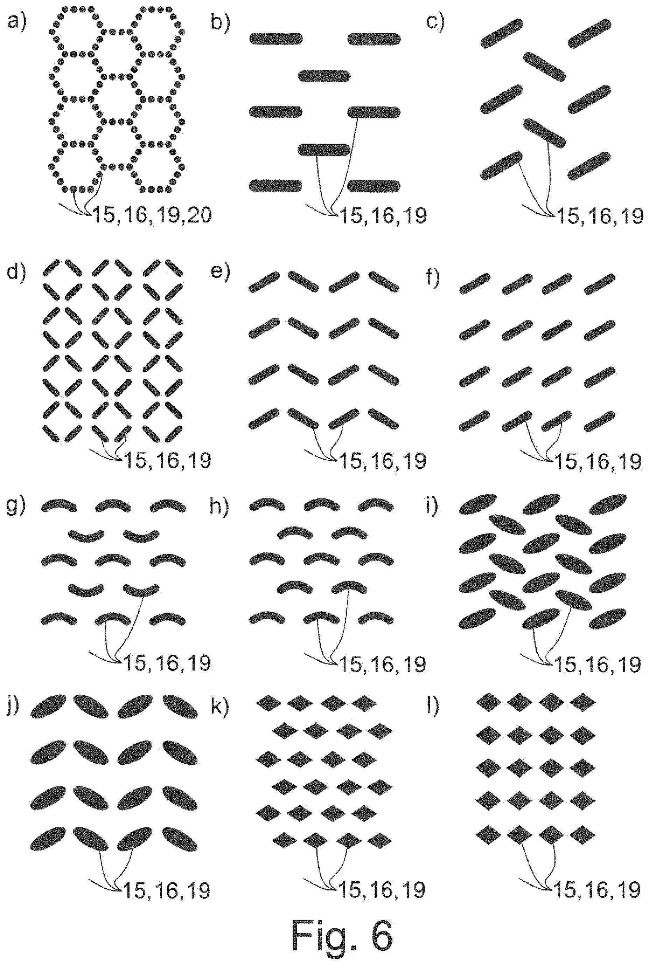

Ebenfalls kann vorgesehen sein, dass einzelne oder alle Prägepunkte 20 jeweils eine punktsymmetrische und/oder achsensymmetrische Querschnittsform aufweisen, und/oder dass einzelne oder alle Prägepunkte 20 jeweils eine runde, eckige und/oder streifenförmige Querschnittsform aufweisen. Mit dem Querschnitt ist jeweils die Draufsicht in Projektion gemeint, also üblicherweise die Form, die sich idealerweise in Draufsicht ergibt. Beispielhafte Bondingmuster mit entsprechenden Bondingmusterelementen 15, gebondeten Bereichen 16 und Prägeelementen 19 bzw. Prägepunkten 20 sind in Fig. 6a)-l) gezeigt. Hier zeigt sich die ganze Vielfalt möglicher Gestaltungen für die Umsetzung der vorschlagsgemäßen Lösung.

Um in Hauptzugrichtung L eine besonders hohe Scherfestigkeit zu erreichen, kann vorgesehen sein, dass die gebondeten Bereiche 16 derart angeordnet sind, dass im Haltezustand mindestens 50% aller an einem gemeinsamen Punkt startenden gedachten Strahlen mit einem maximalen Winkel von 45° zur Längsrichtung R für mindestens 50% aller Punkte der Fläche der Hakenbereiche 11, die einen ungebondeten Bereich 17 überdecken, innerhalb von maximal 8 mm, vorzugsweise 6 mm, weiter vorzugsweise 5 mm, noch weiter vorzugsweise 4 mm, auf einen gebondeten Bereich 16 treffen. Zusätzlich oder alternativ treffen die Strahlen für mindestens 50% aller Punkte der Fläche der Hakenbereiche 11, die einen ungebondeten Bereich 17 überdecken, erst nach mindestens 1 mm, vorzugsweise 2 mm, weiter vorzugsweise 3 mm auf einen gebondeten Bereich 16.

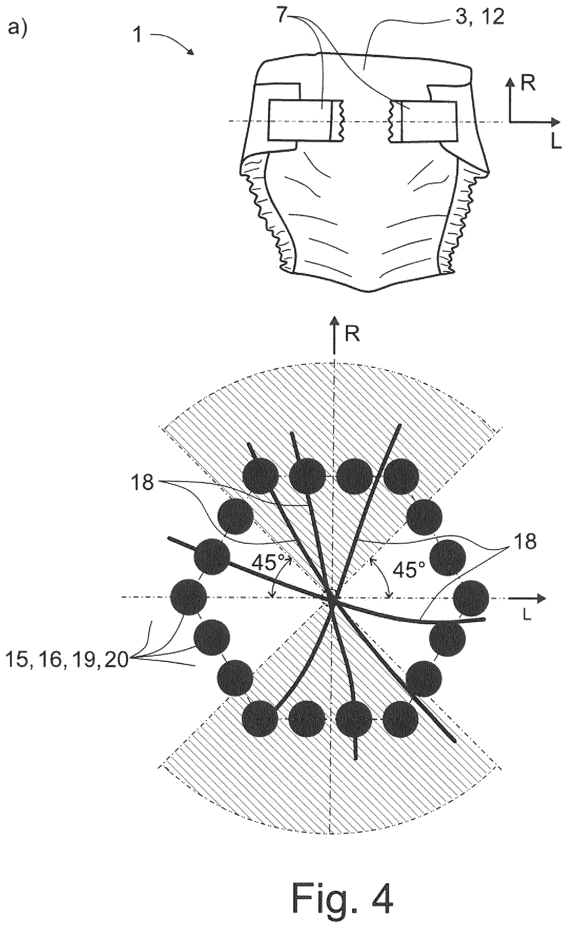

Das obengenannte Prinzip ist in Fig. 4 verdeutlicht. Für einen beliebigen Punkt können Strahlen definiert werden, die in einem maximalen Winkel von 45° in Fig. 4 nach oben und unten zur Längsrichtung R verlaufen. Die Längsrichtung R wiederum verläuft entlang der Körperlängsachse des Benutzers im Stehen also senkrecht zum Boden. Die Längsrichtung R ist hier und vorzugsweise orthogonal zur Hauptzugrichtung L. Somit werden hier die oberen und unteren 90° betrachtet, während die rechten und linken 90° in Fig. 4 vorerst außen vor bleiben. Dies deckt die Richtungen ab, in denen die meisten Fasern 18 der Vliesschicht 12 liegen, die wirksam zur Scherfestigkeit beitragen, da sie Haken 11a quer zur Hauptzugrichtung L halten.

Es ist nun also so, dass unabhängig davon, welcher Punkt eines gebondeten Bereichs betrachtet wird, dieser mit einer mindestens 50%igen Wahrscheinlichkeit so zu den gebondeten Bereichen 16 ausgerichtet ist, dass in mindestens 50% dieser nach oben und unten weisende 90°, zusammen 180°, ein gebondeter Bereich innerhalb der genannten Millimeter-Werte liegt. Somit wird letztendlich eine Wahrscheinlichkeit definiert, dass eine von einem Haken eingehakte Faser 18 ein- oder zweifach innerhalb eines gewissen Abstands gebondet ist, sofern diese Faser quer zur Hauptzugrichtung L verläuft. Dieses quer ist in diesem Fall mit maximal etwa 45° zu betrachten, wobei die Fasern 18 bekanntermaßen relativ chaotisch verlaufen.

Es kann auch vorgesehen sein, dass die gebondeten Bereiche 16 derart angeordnet sind, dass im Haltezustand mindestens 50% aller an einem gemeinsamen Punkt startenden gedachten Strahlen für mindestens 50% aller Punkte der Fläche der Hakenbereiche, die einen ungebondeten Bereich 17 überdecken, innerhalb von maximal 8 mm, vorzugsweise 6 mm, weiter vorzugsweise 5 mm, noch weiter vorzugsweise 4 mm, auf einen gebondeten Bereich treffen. Dabei werden nun also die vollständigen 360° betrachtet. Auch hier können die genannten Minimalwerte des Abstands gelten.

Für alle der jeweils genannten Prozentwerte, die mit 50% angegeben wurden, gelten jeweils oder einzeln vorzugsweise auch Prozentwerte von 75%, vorzugsweise 90%, weiter vorzugsweise 100%.

Alternativ oder zusätzlich kann das Bondingmuster 14 derart ausgestaltet sein, dass die Summe der Längen in Hauptzugrichtung L der Bondingmusterelemente 15 mindestens 70 m pro m² beträgt. Dabei wird die Erstreckung der Bondingmusterelemente 15 zwischen den am weitesten in Hauptzugrichtung L liegenden Enden gemessen. Bei den punktförmigen Bondingmusterelementen 15 ist dies der Durchmesser, bei schräg zur Hauptzugrichtung verlaufenden Linien beispielsweise der in Hauptzugrichtung L verlaufende Anteil des Abstands zwischen den Endpunkten der Linie. Beispielhaft wäre dies der Cosinus des Winkels zwischen der Linie und einer in Hauptzugrichtung L verlaufenden Linie multipliziert mit der Gesamtlänge der Linie. Die Breitenerstreckung in Längsrichtung R der Bondingmusterelemente 15 wird also nicht berücksichtigt. Vorzugsweise beträgt die Summe der Längen in Hauptzugrichtung L der Bondingmusterelemente 15 mindestens 120 m pro m², weiter vorzugsweise mindestens 170 m pro m², noch weiter vorzugsweise mindestens 220 m pro m² und/oder höchstens 500 m pro m², weiter vorzugsweise höchstens 400 m pro m², noch weiter vorzugsweise höchstens 300 m pro m².

Ganz allgemein kann vorgesehen sein, dass die Vliesschicht 12 auf einem Vliesschicht-Trägermaterial, insbesondere einer Folie, aufgebracht ist.

Auch eine spezielle mittlere Faserlänge der die Vliesschicht 12 bildenden Fasern 18 kann bei der vorschlagsgemäßen Windel 1 vorteilhaft sein. So beträgt hier und vorzugsweise die mittlere Faserlänge der Fasern 18 mindestens ein Mehrfaches des minimalen Abstands oder maximalen Abstands der zwei einander diametral gegenüberliegenden Prägeelemente 19 bzw. Prägepunkte 20. Die mittlere Faserlänge kann für die vorschlagsgemäße Windel zwischen 3 und 130 mm liegen. Vorzugsweise liegt die mittlere Faserlänge zwischen 25 und 130 mm, weiter vorzugsweise zwischen 40 und 80 mm, was insbesondere für Fasern 18 eines Spinnvlieses gilt. Im Falle eines Spinnvlieses kann es sich bei den Fasern 18 auch um Endlosfasern handeln. Insbesondere bei Stapelfasern kann die mittlere Faserlänge auch zwischen 3 und 30 mm, weiter vorzugsweise zwischen 5 und 20 mm, liegen.

Weiterhin ist es vorteilhaft, wenn die Faserfeinheit der Fasern 18 zwischen 0,01 und 8 dtex, vorzugsweise zwischen 0,05 und 2,5 dtex, weiter vorzugsweise zwischen 0,5 und 2,5 dtex, weiter weiter vorzugsweise zwischen 1,5 und 2,5 dtex, liegt. Hier handelt es sich bei der Vliesschicht 12 beispielhaft um ein Spinnvlies aus Fasern 18 mit einer Faserfeinheit von 1,7 dtex. Zusätzlich oder alternativ kann die Faserstoffdichte der Vliesschicht 14 zwischen 10 und 20 g/m², vorzugsweise zwischen 12 und 18 g/m², weiter vorzugsweise zwischen 13 und 17 g/m², liegen. Mit der Faserfeinheit ist dabei insbesondere die mittlere Faserfeinheit der die Vliesschicht 12 bildenden Fasern 18 gemeint. Mit der Faserstoffdichte ist entsprechend insbesondere die mittlere Faserstoffdichte der Vliesschicht 12 gemeint.

Auch die Seitenflügel 8 bestehen hier und vorzugsweise aus einem Vlies, wobei alle Ausführungen zur Vliesschicht 12 ebenfalls gelten können. Es kann vorgesehen sein, dass die Befestigungslasche 7 für die Einnahme eines Transportzustands mit der Befestigungsfläche 13 an dem Seitenflügel 8 befestigbar ist.

Die Befestigungslasche 7 weist hier und vorzugsweise im Haltezustand gegenüber dem Rückenblatt 3 und/oder im Transportzustand gegenüber dem Seitenflügel 8 eine maximale Peel-Kraft von 3 N bis 10 N, vorzugsweise 4 N bis 8 N, auf.

Die maximale Peel-Kraft wird vorzugsweise mittels eines T-Peeltests an der Windel 1 gemessen. Der Maximalwert wird dabei durch das Mitteln von zehn Maximalwerten aus zehn Versuchen ermittelt. Ein T-Peeltest ist insofern eine standardmäßige Methode, die entsprechend bekannt ist.

Es kann weiterhin vorgesehen sein, dass der Seitenflügel 8, beispielsweise in dem dieser nicht gebondet wird oder dichter gebondet wird, derart ausgestaltet ist, dass eine Peel-Kraft der Befestigungslasche 7 im Transportzustand geringer ist als im Haltezustand.

## Patentansprüche

1. Wegwerfbare Windel, insbesondere Babywindel, mit einem körperseitigen Deckblatt (2), mit einem körperfernen Rückenblatt (3) und mit mindestens einem Seitenflügel (8), wobei an dem Seitenflügel (8) eine Befestigungslasche (7) angeordnet ist, wobei die Befestigungslasche (7) eine Befestigungsfläche (13) mit Hakenbereichen (11) aufweist,
wobei das Rückenblatt (3) eine Vliesschicht (12) aufweist, wobei die Vliesschicht (12) des Rückenblatts (3) in einem Bondingmuster (14) aus Bondingmusterelementen (15) gebondet ist und so in gebondete Bereiche (16) und ungebondete Bereiche (17) unterteilt ist und
wobei die Befestigungsfläche (13) für die Einnahme eines Haltezustands derart an dem Rückenblatt (3) befestigbar ist, dass die Befestigungsfläche (13) gebondete und ungebondete Bereiche (15, 16) überdeckt,
**dadurch gekennzeichnet,**
**dass** die Befestigungslasche (7) flächig außerhalb der Hakenbereiche (11) höchstens Kleberbereiche (21) aufweist, die im Haltezustand weniger als 20% der von der Befestigungsfläche (13) überdeckten, ungebondeten Bereiche (17) überdecken, und
**dass** mindestens ein hakenfreier Bereich (22) der Befestigungslasche (7), insbesondere ein Kleberbereich (21), zwischen den Hakenbereichen (11) angeordnet ist.

2. Wegwerfbare Windel nach Anspruch 1, **dadurch gekennzeichnet, dass** die Befestigungslasche (7) flächig außerhalb der Hakenbereiche (11) Kleberbereiche (21) aufweist, die im Haltezustand mindestens 1%, vorzugsweise mindestens 4%, weiter vorzugsweise mindestens 8%, der von der Befestigungsfläche (13) überdeckten, ungebondeten Bereiche (17) überdecken, und/oder, dass die Befestigungslasche (7) flächig außerhalb der Hakenbereiche (11) höchstens Kleberbereiche (21) aufweist, die im Haltezustand weniger als 19,5%, vorzugsweise weniger als 19%, weiter vorzugsweise weniger als 17%, noch weiter vorzugsweise weniger als 15%, noch weiter vorzugsweise weniger als 12%, der von der Befestigungsfläche (13) überdeckten, ungebondeten Bereiche (17) überdecken.

3. Wegwerfbare Windel nach Anspruch 1 oder 2, **dadurch gekennzeichnet, dass** die Befestigungslasche (7) mindestens zwei, vorzugsweise mindestens drei, voneinander beabstandete Hakenbereiche (11) aufweist, und/oder, dass die Befestigungslasche (7) mindestens zwei, vorzugsweise mindestens drei, voneinander getrennte Hakenbereiche (11) aufweist.

4. Wegwerfbare Windel nach einem der vorhergehenden Ansprüche, **dadurch gekennzeichnet, dass** mindestens ein Hakenbereich (11), vorzugsweise mindestens zwei Hakenbereiche (11), weiter vorzugsweise mindestens drei Hakenbereiche (11), insbesondere alle Hakenbereiche (11), länglich, vorzugsweise rechteckig, oder quadratisch ist oder sind, und/oder, dass mindestens ein Hakenbereich (11), vorzugsweise mindestens zwei Hakenbereiche (11), weiter vorzugsweise mindestens drei Hakenbereiche (11), insbesondere alle Hakenbereiche (11), eine maximale Breite (B) entlang einer Hauptzugrichtung (L) von 8 mm, vorzugsweise 6 mm, weiter vorzugsweise 4 mm, aufweist oder aufweisen, und/oder, dass mindestens ein Hakenbereich (11), vorzugsweise mindestens zwei Hakenbereiche (11), weiter vorzugsweise mindestens drei Hakenbereiche (11), insbesondere alle Hakenbereiche (11), eine minimale Breite (B) entlang der Hauptzugrichtung (L) von 2 mm, vorzugsweise 3 mm, weiter vorzugsweise 4 mm, aufweist oder aufweisen.

5. Wegwerfbare Windel nach einem der vorhergehenden Ansprüche, **dadurch gekennzeichnet, dass** mindestens zwei, vorzugsweise mindestens drei, hakenfreie Bereiche (22) der Befestigungslasche (7) zwischen den Hakenbereichen (11) angeordnet sind, und/oder, dass mindestens einer, vorzugsweise mindestens zwei, der zwischen den Hakenbereichen (11) angeordneten hakenfreien Bereiche (22) vollständig von Hakenbereichen (11) umgeben ist oder sind und/oder mindestens einer, vorzugsweise mindestens zwei, der zwischen den Hakenbereichen (11) angeordneten hakenfreien Bereiche (22) auf mindestens einer, vorzugsweise mindestens zwei, Seiten nicht an einen Hakenbereich (11) grenzt.

6. Wegwerfbare Windel nach einem der vorhergehenden Ansprüche, **dadurch gekennzeichnet, dass** mindestens ein hakenfreier Bereich (22), vorzugsweise mindestens zwei hakenfreie Bereiche (22), weiter vorzugsweise mindestens drei hakenfreie Bereiche (22), insbesondere alle hakenfreien, Bereiche (22), länglich, vorzugsweise rechteckig, oder quadratisch ist oder sind, und/oder, dass mindestens ein hakenfreier Bereich (22), vorzugsweise mindestens zwei hakenfreie Bereiche (22), weiter vorzugsweise mindestens drei hakenfreie (22) Bereiche, insbesondere alle hakenfreien Bereiche (22), eine maximale Breite (C) entlang der Hauptzugrichtung (L) von 6 mm, vorzugsweise 4 mm, weiter vorzugsweise 2 mm, aufweist oder aufweisen, und/oder, dass mindestens ein hakenfreier Bereich (22), vorzugsweise mindestens zwei hakenfreie Bereiche (22), weiter vorzugsweise mindestens drei hakenfreie Bereiche (22), insbesondere alle hakenfreien Bereiche (22), eine minimale Breite (C) entlang der Hauptzugrichtung (L) von 0,5 mm, vorzugsweise 1 mm, weiter vorzugsweise 1,5 mm, aufweist oder aufweisen.

7. Wegwerfbare Windel nach einem der vorhergehenden Ansprüche, **dadurch gekennzeichnet, dass** die Befestigungsfläche (13) und/oder die Hakenbereiche (11) eine Gesamtbreite entlang der Hauptzugrichtung (L) von mindestens 0,7 cm, vorzugsweise mindestens 1 cm, weiter vorzugsweise mindestens 1,2 cm und/oder eine maximale Gesamtbreite entlang der Hauptzugrichtung (L) von 2 cm, vorzugsweise 1, 7 cm, weiter vorzugsweise 1,5 cm aufweist oder aufweisen.

8. Wegwerfbare Windel nach einem der vorhergehenden Ansprüche, **dadurch gekennzeichnet, dass** die Hakenbereiche (11) eine Flächendichte an Haken (11a) von mindestens 150 Haken pro 100 mm², vorzugsweise mindestens 200 Haken pro 100 mm², weiter vorzugsweise mindestens 250 Haken pro 100 mm², und/oder höchstens 450 Haken pro 100 mm², vorzugsweise höchstens 400 Haken pro 100 mm², weiter vorzugsweise höchstens 350 Haken pro 100 mm², aufweisen.

9. Wegwerfbare Windel nach einem der vorhergehenden Ansprüche, **dadurch gekennzeichnet, dass** das Bondingmuster (14) zumindest den größten Teil der Fläche der Vliesschicht (12), vorzugsweise die gesamte Fläche der Vliesschicht (12), einnimmt, und/oder, dass die Vliesschicht (12) zumindest den größten Teil der Fläche des Rückenblatts (3), insbesondere die gesamte Fläche des Rückenblattes (3), einnimmt.

10. Wegwerfbare Windel nach einem der vorhergehenden Ansprüche, **dadurch gekennzeichnet, dass** die Bondingmusterelemente (15) so angeordnet sind, dass die ungebondeten Bereiche (17) regelmäßige Musterelemente ergeben, vorzugsweise, dass die Musterelemente ohne Zwischenräume angeordnet sind und/oder alle Musterelemente denselben Querschnitt aufweisen.

11. Wegwerfbare Windel nach Anspruch 10, **dadurch gekennzeichnet, dass** einzelne oder alle Musterelemente jeweils eine punktsymmetrische und/oder achsensymmetrische Außenkontur aufweisen, und/oder, dass einzelne oder alle Musterelemente jeweils eine runde und/oder eckige, vorzugsweise hexagonale, Außenkontur aufweisen, vorzugsweise, dass das Bondingmuster (14) eine Hexagonalstruktur aufweist.

12. Wegwerfbare Windel nach Anspruch 10 oder 11, **dadurch gekennzeichnet, dass** die Bondingmusterelemente (15) Prägeelemente (19), insbesondere zueinander, insbesondere gleichmäßig, beabstandete Prägepunkte (20) und/oder Prägelinien aufweisen, wobei im Bereich des jeweiligen Prägeelements (19) die Vliesschicht (12) ihre minimale Dicke aufweist, vorzugsweise, dass einzelne oder alle Prägepunkte (20) jeweils eine punktsymmetrische und/oder achsensymmetrische Querschnittsform aufweisen, und/oder, dass einzelne oder alle Prägepunkte (20) jeweils eine runde, eckige und/oder streifenförmige Querschnittsform aufweisen.

13. Wegwerfbare Windel nach einem der vorhergehenden Ansprüche, **dadurch gekennzeichnet, dass** die gebondeten Bereiche (16) derart angeordnet sind, dass im Haltezustand mindestens 50% aller an einem gemeinsamen Punkt startenden gedachten Strahlen mit einem maximalen Winkel von 45° zur Längsrichtung (R) für mindestens 50% aller Punkte der Fläche der Hakenbereiche (11), die einen ungebondeten Bereich (17) überdecken, innerhalb von maximal 8 mm, vorzugsweise 6 mm, weiter vorzugsweise 5 mm, noch weiter vorzugsweise 4 mm, auf einen gebondeten Bereich (16) treffen.

14. Wegwerfbare Windel nach einem der vorhergehenden Ansprüche, **dadurch gekennzeichnet, dass** die gebondeten Bereiche (16) derart angeordnet sind, dass im Haltezustand mindestens 50% aller an einem gemeinsamen Punkt startenden gedachten Strahlen für mindestens 50% aller Punkte der Fläche der Hakenbereiche (11), die einen ungebondeten Bereich (17) überdecken, innerhalb von maximal 8 mm, vorzugsweise 6 mm, weiter vorzugsweise 5 mm, noch weiter vorzugsweise 4 mm, auf einen gebondeten Bereich (16) treffen.

15. Wegwerfbare Windel nach einem der vorhergehenden Ansprüche, **dadurch gekennzeichnet, dass** die Vliesschicht (12) auf einem Vliesschicht-Trägermaterial, insbesondere einer Folie, aufgebracht ist.

16. Wegwerfbare Windel nach einem der vorhergehenden Ansprüche, **dadurch gekennzeichnet, dass** die mittlere Faserlänge der die Vliesschicht (12) bildenden Fasern (18) zwischen 3 und 130 mm liegt, vorzugsweise, dass die mittlere Faserlänge der die Vliesschicht (12) bildenden Fasern (18) zwischen 30 und 130 mm, weiter vorzugsweise zwischen 40 und 80 mm, liegt oder dass die mittlere Faserlänge der die Vliesschicht (12) bildenden Fasern (18) zwischen 3 und 30 mm, weiter vorzugsweise zwischen 5 und 20 mm, liegt.

17. Wegwerfbare Windel nach einem der vorhergehenden Ansprüche, **dadurch gekennzeichnet, dass** die Faserfeinheit der die Vliesschicht (12) bildenden Fasern (18) zwischen 0,01 und 8 dtex, vorzugsweise zwischen 0,05 und 2,5 dtex, weiter vorzugsweise zwischen 0,5 und 2,5 dtex, weiter vorzugsweise zwischen 1,5 und 2,5 dtex, liegt, und/oder, dass die Faserstoffdichte der Vliesschicht (12) zwischen 10 und 20 g/m², vorzugsweise zwischen 12 und 18 g/m², weiter vorzugsweise zwischen 13 und 17 g/m², liegt.

18. Wegwerfbare Windel nach einem der vorhergehenden Ansprüche, **dadurch gekennzeichnet, dass** die Befestigungslasche (7) im Haltezustand eine maximale Peel-Kraft von 3 N bis 10 N, vorzugsweise 4 N bis 8 N, gegenüber dem Rückenblatt (3) und/oder dem der Befestigungslasche (7) zugeordneten Seitenflügel (8) aufweist.

## Claims

1. Disposable diaper, in particular baby diaper, having a body-proximal cover sheet (2), having a body-distal back sheet (3), and having at least one side flap (8), wherein a fastening tab (7) is disposed on the side flap (8), wherein the fastening tab (7) has a fastening area (13) having hook regions (11);
wherein the back sheet (3) has a non-woven layer (12), wherein the non-woven layer (12) of the back sheet (3) is bonded in a bonding pattern (14) of bonding pattern elements (15), and thus is subdivided into bonded regions (16) and non-bonded regions (17); and
wherein the fastening area (13) for assuming a holding state is able to be fastened to the back sheet (3) in such a manner that the fastening area (13) covers bonded and non-bonded regions (15, 16), **characterized in that**
the fastening tab (7) in terms of area outside the hook regions (11) has at most adhesive regions (21) which in the holding state cover less than 20% of the non-bonded regions (17) covered by the fastening area (13); and
**in that** at least one hook-free region (22) of the fastening tab (7), in particular an adhesive region (21), is disposed between the hook regions (11).

2. Disposable diaper according to Claim 1, **characterized in that** the fastening tab (7) in terms of area outside the hook regions (11) has adhesive regions (21) which in the holding state cover at least 1%, preferably at least 4%, furthermore preferably at least 8%, of the non-bonded regions (17) covered by the fastening area (13), and/or **in that** the fastening tab (7) in terms of area outside the hook regions (11) has at most adhesive regions (21) which in the holding state cover less than 19.5%, preferably less than 19%, furthermore preferably less than 17%, even furthermore preferably less than 15%, even furthermore preferably less than 12%, of the non-bonded regions (17) covered by the fastening area (13).

3. Disposable diaper according to Claim 1 or 2, **characterized in that** the fastening tab (7) has at least two, preferably at least three, mutually spaced apart hook regions (11), and/or **in that** the fastening tab (7) has at least two, preferably at least three, mutually separate hook regions (11).

4. Disposable diaper according to one of the preceding claims, **characterized in that** at least one hook region (11), preferably at least two hook regions (11), furthermore preferably at least three hook regions (11), in particular all hook regions (11), is/are elongate, preferably rectangular or square, and/or **in that** at least one hook region (11), preferably at least two hook regions (11), furthermore preferably at least three hook regions (11), in particular all hook regions (11), has/have a maximum width (B) along a main tensile direction (L) of 8 mm, preferably 6 mm, furthermore preferably 4 mm, and/or **in that** at least one hook region (11), preferably at least two hook regions (11), furthermore preferably at least three hook regions (11), in particular all hook regions (11), has/have a minimum width (B) along the main tensile direction (L) of 2 mm, preferably 3 mm, furthermore preferably 4 mm.

5. Disposable diaper according to one of the preceding claims, **characterized in that** at least two, preferably at least three, hook-free regions (22) of the fastening tab (7) are disposed between the hook regions (11), and/or **in that** at least one, preferably at least two, of the hook-free regions (22) disposed between the hook regions (11) is/are completely surrounded by hook regions (11) and/or at least one, preferably at least two, of the hook-free regions (22) disposed between the hook regions (11) is/are not contiguous to a hook region (11) on at least one side, preferably at least two sides.

6. Disposable diaper according to one of the preceding claims, **characterized in that** at least one hook-free region (22), preferably at least two hook-free regions (22), furthermore preferably at least three hook-free regions (22), in particular all hook-free regions (22), is/are elongate, preferably rectangular, or square, and/or **in that** at least one hook-free region (22), preferably at least two hook-free regions (22), furthermore preferably at least three hook-free regions (22), in particular all hook-free regions (22), has/have a maximum width (C) along the main tensile direction (L) of 6 mm, preferably 4 mm, furthermore preferably 2 mm, and/or **in that** at least one hook-free region (22), preferably at least two hook-free regions (22), furthermore preferably at least three hook-free regions (22), in particular all hook-free regions (22) has/have a minimum width (C) along the main tensile direction (L) of 0.5 mm, preferably 1 mm, furthermore preferably 1.5 mm.

7. Disposable diaper according to one of the preceding claims, **characterized in that** the fastening area (13) and/or the hook regions (11) has/have an overall width along the main tensile direction (L) of at least 0.7 cm, preferably at least 1 cm, furthermore preferably at least 1.2 cm and/or a maximum overall width along the main tensile direction (L) of 2 cm, preferably 1.7 cm, furthermore preferably 1.5 cm.

8. Disposable diaper according to one of the preceding claims, **characterized in that** the hook regions (11) have an area density in terms of hooks (11a) of at least 150 hooks per 100 mm², preferably at least 200 hooks per 100 mm², furthermore preferably at least 250 hooks per 100 mm² and/or at most 450 hooks per 100 mm², preferably at most 400 hooks per 100 mm², furthermore preferably at most 350 hooks per 100 mm².

9. Disposable diaper according to one of the preceding claims, **characterized in that** the bonding pattern (14) occupies at least the majority of the area of the non-woven layer (12), preferably the entire area of the non-woven layer (12), and/or **in that** the non-woven layer (12) occupies at least the majority of the area of the back sheet (3), in particular the entire area of the back sheet (3).

10. Disposable diaper according to one of the preceding claims, **characterized in that** the bonding pattern elements (15) are disposed in such a way that the non-bonded regions (17) result in regular pattern elements, preferably that the pattern elements are disposed without intermediate spaces and/or all pattern elements have the same cross section.

11. Disposable diaper according to Claim 10, **characterized in that** individual or all pattern elements have in each case an external contour with a symmetry in respect of a point and/or axis, and/or **in that** individual or all pattern elements have in each case a round and/or angular, preferably hexagonal, external contour, preferably that the bonding pattern (14) has a hexagonal structure.

12. Disposable diaper according to Claim 10 or 11, **characterized in that** the bonding pattern elements (15) have embossed elements (19), in particular embossed points (20) and/or embossed lines which are, in particular uniformly, spaced apart from one another, wherein the non-woven layer (12) has its minimum thickness in the region of the respective embossed element (19), preferably **in that** individual or all embossed points (20) have in each case a cross-sectional shape with a symmetry in respect of a point and/or axis, and/or **in that** individual or all embossed points (20) have in each case a round, angular and/or stripe-shaped cross-sectional shape.

13. Disposable diaper according to one of the preceding claims, **characterized in that** the bonded regions (16) are disposed in such a manner that in the holding state at least 50% of all imaginary rays starting at a common point, for at least 50% of all points of the area of the hook regions (11) that cover a non-bonded region (17), meet a bonded region (16) at a maximum angle of 45° in relation to the longitudinal direction (R) within at most 8 mm, preferably 6 mm, furthermore preferably 5 mm, even furthermore preferably 4 mm.

14. Disposable diaper according to one of the preceding claims, **characterized in that** the bonded regions (16) are disposed in such a manner that in the holding state at least 50% of all imaginary rays starting at a common point, for at least 50% of all points of the area of the hook regions (11) that cover a non-bonded region (17), meet a bonded region (16) within at most 8 mm, preferably 6 mm, furthermore preferably 5 mm, even furthermore preferably 4 mm.

15. Disposable diaper according to one of the preceding claims, **characterized in that** the non-woven layer (12) is applied to a non-woven carrier material, in particular a film.

16. Disposable diaper according to one of the preceding claims, **characterized in that** the mean fibre length of the fibres (18) forming the non-woven layer (12) is between 3 and 130 mm, preferably **in that** the mean fibre length of the fibres (18) forming the non-woven layer (12) is between 30 and 130 mm, furthermore preferably between 40 and 80 mm, or **in that** the mean fibre length of the fibres (18) forming the non-woven layer (12) is between 3 and 30 mm, furthermore preferably between 5 and 20 mm.

17. Disposable diaper according to one of the preceding claims, **characterized in that** the fibre fineness of the fibres (18) forming the non-woven layer (12) is between 0.01 and 8 dtex, preferably between 0.05 and 2.5 dtex, furthermore preferably between 0.5 and 2.5 dtex, furthermore preferably between 1.5 and 2.5 dtex, and/or in that the fibrous density of the non-woven layer (12) is between 10 and 20 g/m², preferably between 12 and 18 g/m², furthermore preferably between 13 and 17 g/m².

18. Disposable diaper according to one of the preceding claims, **characterized in that** the fastening tab (7) in the holding state has a maximum peel force of 3 N to 10 N, preferably 4 N to 8 N, in comparison to the back sheet (3) and/or the side flap (8) assigned to the fastening tab (7).

## Revendications

1. Couche jetable, en particulier couche pour bébé, comprenant une feuille de recouvrement (2) du côté du corps, une feuille arrière (3) du côté éloigné du corps et au moins une patte latérale (8), une languette de fixation (7) étant disposée sur la patte latérale (8), la languette de fixation (7) comportant une surface de fixation (13) pourvue de zones de crochet (11),
la feuille arrière (3) comportant une couche de non-tissé (12),
la couche de non-tissé (12) de la feuille arrière (3) étant liée suivant un motif de liaison (14) formé d'éléments de motif de liaison (15) et étant ainsi divisée en zones liées (16) et en zones non liées (17) et
la surface de fixation (13) pouvant être fixée à la feuille arrière (3) afin de prendre un état de retenue de manière à ce que la surface de fixation (13) recouvre des zones liées et non liées (15, 16), **caractérisée en ce que**
la languette de fixation (7) comporte de manière sensiblement bidimensionnelle à l'extérieur des zones à crochets (11) au plus des zones adhésives (21) qui, à l'état de retenue, recouvrent moins de 20 % des zones non liées (17) recouvertes par la surface de fixation (13), et
au moins une zone sans crochets (22) de la languette de fixation (7), en particulier une zone adhésive (21), est disposée entre les zones à crochets (11).

2. Couche jetable selon la revendication 1, **caractérisée en ce que** la languette de fixation (7) comporte de manière sensiblement bidimensionnelle, à l'extérieur des zones à crochets (11), des zones adhésives (21) qui, à l'état de retenue, recouvrent au moins 1 %, de préférence au moins 4 %, plus préférablement au moins 8 %, des zones non liées (17) recouvertes par la surface de fixation (13), et/ou **en ce que** la languette de fixation (7) comporte de manière sensiblement bidimensionnelle, à l'extérieur des zones à crochets (11), au plus des zones adhésives (21) qui, à l'état de retenue, recouvrent moins de 19,5 %, de préférence moins de 19 %, plus préférablement moins de 17 %, encore plus préférablement moins de 15%, encore plus préférablement moins de 12 %, de la surface de fixation (13) recouverte par des zones non liées (17).

3. Couche jetable selon la revendication 1 ou 2, **caractérisée en ce que** la languette de fixation (7) comporte au moins deux, de préférence au moins trois, zones à crochets (11) espacées les unes des autres et/ou **en ce que** la languette de fixation (7) comporte au moins deux, de préférence au moins trois, zones à crochet (11) séparées les unes des autres.

4. Couche jetable selon l'une des revendications précédentes, **caractérisée en ce qu'**au moins une zone à crochets (11), de préférence au moins deux zones à crochets (11), plus préférablement au moins trois zones à crochets (11), en particulier toutes les zones à crochets (11), sont allongées, de préférence rectangulaire, ou carrées, et/ou **en ce qu'**au moins une zone à crochets (11), de préférence au moins deux zones à crochets (11), plus préférablement au moins trois zones à crochets (11), en particulier toutes les zones à crochets (11), ont suivant une direction de traction principale (L) une largeur maximale (B) de 8 mm, de préférence 6 mm, plus préférablement de 4 mm, et/ou **en ce qu'**au moins une zone à crochets (11), de préférence au moins deux zones à crochets (11), plus préférablement au moins trois zones à crochet (11), en particulier toutes les zones à crochets (11) ont suivant la direction de traction principale (L) une largeur minimale (B) de 2 mm, de préférence de 3 mm, plus préférablement de 4 mm.

5. Couche jetable selon l'une des revendications précédentes, **caractérisée en ce qu'**au moins deux, de préférence au moins trois, zones sans crochets (22) de la languette de fixation (7) sont disposées entre les zones à crochets (11), et/ou **en ce qu'**au moins une, de préférence au moins deux, des zones sans crochets (22) disposées entre les zones à crochets (11) sont entièrement entourées par des zones à crochets (11) et/ou au moins une, de préférence au moins deux, des zones sans crochets (22) disposées entre les zones à crochets (11) ne bordent pas une zone à crochets (11) sur au moins un côté, de préférence au moins deux côtés.

6. Couche jetable selon l'une des revendications précédentes, **caractérisée en ce qu'**au moins une zone sans crochets (22), de préférence au moins deux zones sans crochets (22), plus préférablement au moins trois zones sans crochets (22), en particulier toutes les zones sans crochets (22), sont allongées, de préférence rectangulaires ou carrées, et/ou **en ce qu'**au moins une zone sans crochets (22), de préférence au moins deux zones sans crochets (22), plus préférablement au moins trois zones sans crochets (22), en particulier, toutes les zones sans crochets (22) ont suivant la direction de traction principale (L) une largeur maximale (C) de 6 mm, de préférence 4 mm, plus préférablement 2 mm, et/ou **en ce qu'**au moins une zone sans crochets (22), de préférence au moins deux zones sans crochets (22), plus préférablement au moins trois zones sans crochets (22), en particulier toutes les zones sans crochets (22) ont suivant la direction de traction principale (L) une largeur minimale (C) de 0,5 mm, de préférence 1 mm, plus préférablement 1,5 mm.

7. Couche jetable selon l'une des revendications précédentes, **caractérisée en ce que** la surface de fixation (13) et/ou les zones à crochets (11) ont suivant la direction de traction principale (L) une largeur totale d'au moins 0,7 cm, de préférence d'au moins 1 cm, plus préférablement d'au moins 1,2 cm et/ou suivant la direction de traction principale (L) une largeur totale maximale de 2 cm, de préférence 1,7 cm, plus préférablement 1,5 cm.

8. Couche jetable selon l'une des revendications précédentes, **caractérisée en ce que** les zones à crochets (11) ont une densité surfacique de crochets (11a) d'au moins 150 crochets pour 100 mm², de préférence d'au moins 200 crochets pour 100 mm², plus préférablement au moins 250 crochets pour 100 mm², et/ou au plus 450 crochets pour 100 mm², de préférence au plus 400 crochets pour 100 mm², plus préférablement au plus 350 crochets pour 100 mm².

9. Couche jetable selon l'une des revendications précédentes, **caractérisée en ce que** le motif de liaison (14) occupe au moins la plus grande partie de la surface de la couche de non-tissé (12), de préférence toute la surface de la couche de non-tissé (12), et/ou en ce que la couche de non-tissé (12) occupe au moins la plus grande partie de la surface de la feuille arrière (3), notamment toute la surface de la feuille arrière (3).

10. Couche jetable selon l'une des revendications précédentes, **caractérisée en ce que** les éléments de motif de liaison (15) sont disposés de manière à ce que les zones non liées (17) produisent des éléments de motif réguliers, de préférence **en ce que** les éléments de motif soient disposés sans interstices et/ou tous les éléments de motif aient la même section transversale.

11. Couche jetable selon la revendication 10, **caractérisée en ce que** certains ou tous les éléments de motif ont chacun un contour extérieur à symétrie ponctuelle et/ou à symétrie axiale, et/ou **en ce que** certains ou tous les éléments de motif ont chacun un contour rond et/ou angulaire, de préférence un contour extérieur hexagonal, de préférence **en ce que** le motif de liaison (14) présente une structure hexagonale.

12. Couche jetable selon la revendication 10 ou 11, **caractérisée en ce que** les éléments de motif de liaison (15) présentent des éléments de gaufrage (19), notamment des points de gaufrage (20) et/ou des lignes de gaufrage espacés régulièrement, la couche de non-tissé (12) ayant dans la zone de l'élément de gaufrage (19) respectif son épaisseur minimale, de préférence **en ce que** certains ou tous les points de gaufrage (20) présentent chacun une forme en coupe transversale à symétrie ponctuelle et/ou à symétrie axiale et/ou **en ce que** certains ou tous les points de gaufrage (20) présentent chacun une forme en coupe transversale ronde, carrée et/ou en bande.

13. Couche jetable selon l'une des revendications précédentes, **caractérisée en ce que** les zones liées (16) sont disposées de manière à ce que, à l'état de retenue, au moins 50 % de tous les rayons imaginaires partant d'un point commun soient incidents à une zone liée (16) avec un angle maximum de 45° par rapport à la direction longitudinale (R) pour au moins 50 % de tous les points de la surface des zones à crochets (11) qui recouvrent une zone non liée (17), sur un maximum de 8 mm, de préférence 6 mm, plus préférablement de 5 mm, plus préférablement encore de 4 mm.

14. Couche jetable selon l'une des revendications précédentes, **caractérisée en ce que** les zones liées (16) sont disposées de manière à ce que, à l'état de retenue, au moins 50 % de tous les rayons imaginaires partant d'un point commun soient incidents à une zone liée (16) pour au moins 50 % de tous les points de la surface des zones à crochets (11) qui recouvrent une zone non liée (17), sur un maximum de 8 mm, de préférence 6 mm, plus préférablement de 5 mm, plus préférablement encore de 4 mm.

15. Couche jetable selon l'une des revendications précédentes, **caractérisée en ce que** la couche de non-tissé (12) est appliquée sur un matériau de support de couche de non-tissé, notamment un film.

16. Couche jetable selon l'une des revendications précédentes, **caractérisée en ce que** la longueur moyenne des fibres (18) qui forment la couche de non-tissé (12) est comprise entre 3 et 130 mm, de préférence **en ce que** la longueur moyenne des fibres (18) qui forment la couche de non-tissé (12) est comprise entre 30 et 130 mm, plus préférablement entre 40 et 80 mm, ou **en ce que** la longueur moyenne des fibres (18) qui forment la couche de non-tissé (12) est comprise entre 3 et 30 mm, plus préférablement entre 5 et 20 mm.

17. Couche jetable selon l'une des revendications précédentes, **caractérisée en ce que** la finesse des fibres (18) qui forment la couche de non-tissé (12) est comprise entre 0,01 et 8 dtex, de préférence entre 0,05 et 2,5 dtex, plus préférablement entre 0.5 et 2,5 dtex, plus préférablement entre 1,5 et 2,5 dtex, et/ou **en ce que** la densité des fibres de la couche de non-tissé (12) est comprise entre 10 et 20 g/m², de préférence entre 12 et 18 g/m², plus préférablement entre 13 et 17 g/m².

18. Couche jetable selon l'une des revendications précédentes, **caractérisée en ce que** la languette de fixation (7) présente à l'état de retenue une force de pelage maximale de 3 N à 10 N, de préférence de 4 N à 8 N, par rapport à la feuille arrière (3) et/ou à la patte latérale (8) associée à la languette de fixation (7).
